(19) **Europäisches Patentamt European Patent Office Office européen des brevets**

(11) **EP 4 787 387 A1**

(12) **EUROPEAN PATENT APPLICATION**

(43) Date of publication:
05.08.2026 Bulletin 2026/32

(21) Application number: 26153848.2

(22) Date of filing: 23.01.2026

(51) International Patent Classification (IPC):
*G16H 50/70* *(2018.01)*

(52) Cooperative Patent Classification (CPC):
G16H 50/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 03.02.2025 US 202519044463

(71) Applicant: Optellum Limited
Oxford, Oxfordshire OX1 4EH (GB)

(72) Inventors:
• **Whitley, Conor Alan**
  **Oxford, OX1 4EH (GB)**
• **Ellis, Barnaby George**
  **Oxford, OX1 4EH (GB)**
• **Chometon, Quentin Pierre**
  **Oxford, OX1 4EH (GB)**
• **Montilva, Carlos Federico Arteta**
  **Oxford, OX1 4EH (GB)**

(74) Representative: **Optimus Patents Limited**
**Peak Hill House**
**Steventon**
**Basingstoke, Hampshire RG25 3AZ (GB)**

(54) **SYSTEM AND METHOD FOR LANGUAGE AGNOSTIC PATIENT DISCOVERY**

(57)    A patient discovery system for analysing a database of clinical data associated with a plurality of patients; comprising: a language adaptor circuit to receive a discovery configuration in a query language, the discovery configuration is used to determine patients to be flagged for investigation; a data transformation circuit to receive clinical text and produce transformed clinical data for the patients; wherein the clinical text is in source languages different to the query language; wherein the language adaptor circuit is agnostic to the source and query languages, and receives transformed clinical data, and detects the source language of transformed data, and uses received transformed data and discovery configurations to produce a coupled representation; and a patient discovery circuit for receiving the representation, and determining from the representation if the clinical text meets requirements of the discovery configuration, and if the requirements are met the patient is added to a flag list.

## General overview

FIG 1

EP 4 787 387 A1

# EP 4 787 387 A1

## Description

## Field of Invention

This invention relates to the field of medical informatics and the optimization of clinical workflows using automated systems.

## Background of Invention

In modern healthcare systems, radiological examinations generate vast amounts of textual reports describing findings from various imaging modalities such as x-ray, computerized tomography (CT), magnetic resonance imaging (MRI) and ultrasound. These reports, written by radiologists, contain crucial information about pathological conditions, anatomical features, and other clinically significant findings. However, the efficient extraction and analysis of specific information from these reports present several significant challenges in current clinical practice.

Healthcare institutions worldwide maintain extensive repositories of text-based clinical documentation, including but not limited to medical records, clinical notes, imaging reports, and treatment summaries. This documentation can serve as a rich source of information when communicated and used effectively. For example, radiology reports from imaging studies often contain crucial information regarding incidental findings, disease progression and follow-up recommendations. They can also be used downstream to identify specific cohorts, such as patients who should be closely monitored for lung cancer on the basis of a CT exam.

The extraction of information from text data, despite its typically rich content, has historically been dominated by manual review approaches that carry substantial drawbacks. These traditional methods consume significant time and resources and are prone to human error and biases in interpretation.

Patients with a reported incidental finding are particularly vulnerable to human error and biases. Incidental findings are those discovered opportunistically during imaging studies and other diagnostic tests ordered for a different clinical problem. A systematic review revealed that 23.6% of diagnostic tests lead to an incidental finding, rising to 31.3% for CT imaging studies [1]. These incidental findings often risk being lost to follow-up because the ordering physician typically focuses on the primary indication for the study, neglecting additional findings.

Natural language processing (NLP), a field related to Artificial Intelligence (AI), is being increasingly applied to automatically process clinical text data such as radiology reports and clinical notes. The complexity of processing clinical text data is compounded by their highly specialized medical terminology, varying reporting styles among different clinicians, and differences in language across multiple institutions. Furthermore, despite the rapid progress of deep learning and large language models (LLMs) over the last decade, a systematic review from 2021 [2] found that <10% of radiology report NLP publications use modern deep learning, with the remainder using rule based methods or traditional machine learning. Rule based methods refer to heuristic, deterministic models such as those based on regular expressions. Traditional machine learning typically involves training a model using hand-crafted language features as input. These methods are inherently language specific, limiting their deployment in health system that use a different language, or multi-lingual environments.

Existing approaches to radiology report analysis have primarily focused on natural language processing (NLP) techniques for information extraction. However, these methods often struggle with the variability in report structure, terminology, and language differences. Some systems have attempted to address these challenges by using structured reporting templates or standardized terminologies like RadLex, but adoption has been limited due to workflow disruptions and the preference for free-text reporting among radiologists.

Modem NLP systems embed text into mathematical representations to facilitate their processing within machine learning models. These representations are sometimes referred to as embeddings which are generated in the context of an embedding space, a mathematical abstraction that preserves a relation to the sematic meaning of the objects found in it. Embeddings typically take the shape of n-dimensional vectors, also referred to as feature vectors, each generated for each sub-element of the input string denoted as "token", which can be full words, or any arbitrary splitting of natural text such as statistically based on their occurrence in a given language, and including all possible characters. Algorithms performing this conversion of text strings into sequences of feature vectors in an embedding spacing are referred to as tokenizers.

An example class of NLP systems relying on embedding text is "word embeddings". Word embeddings are high-dimensional vector representations of words, where semantically similar words and synonyms are positioned closer together. These embeddings are typically learned by neural network architectures which determine these representations by analysing how words co-occur in large text corpora - the underlying principle being that words appearing in similar contexts likely have related meanings. These embeddings can be pre-trained on large datasets and used as a basis for a plurality of NLP tasks, where they serve as dense, meaningful input features. A crucial property of word embeddings is that they enable mathematical operations on words, allowing models to understand and work with relationships between different terms in a quantifiable way.

**EP 4 787 387 A1**

State of the art NLP models are referred to as Large Language Models (LLMs), are a class of neural network models, which contain a very large number of parameters (typically > 1 billion). LLMs can build rich and deep understanding of human language by learning patterns from vast text datasets. They operate by first converting input text into a sequence of semantically rich embedding vectors for each token, where a token is an individual word, sub-word or character. These models are typically built on the transformer architecture, which is characterised by the attention mechanism. In a series of 'attention layers', each token's embedding is enriched with information from other tokens in the sequence. This process is often followed by a feed-forward neural network, allowing the model to learn richer representations through non-linear transformations. The resultant token representations are now both semantically and contextually rich, providing a strong basis for downstream tasks such as language generation, translation, sequence classification and question answering.

LLMs have driven substantial progress in NLP in recent years, particularly in domains such as machine translation. The machine translation task involves converting a text sequence from a source language into a target language. One common approach is to use an encoder-decoder architecture: the encoder first transforms the input sequence into high-dimensional, contextualized representations, and the decoder then generates the translation by predicting each subsequent word. An alternative and increasingly popular approach is to use a decoder-only model, which accepts a natural language prompt containing instructions and the text to be translated, then sequentially predicts each word of the final translation.

Automated tools which use NLP to augment and streamline the patient management process are commercially available and used in clinical practice. Optellum's Virtual Nodule Clinic (VNC) [3] offers a functionality called Patient Discovery, which periodically searches incoming radiology reports to identify patients with at least one lung nodule, prompting the clinician (a pulmonologist) or clinical team to decide the appropriate course of action. Similarly, Eon's Patient Management (EPM) software [4] uses NLP to identify actionable findings for several conditions such as incidental pulmonary nodules and liver lesions. These tools are limited to operate in the language for which the model was specifically developed. This would pose numerous challenges if, for example, a tool was deployed at a healthcare provider which spans several nations with different languages. One solution to this would be to have language specific models which serve each language separately. This however may raise issues related to fairness, where models trained on abundant languages achieve a higher performance than scarcer languages. Maintenance, testing and regulation of tools adopting this pluralistic approach may also pose scalability challenges.

<u>References</u>

[1] Lumbreras, B., Donat, L., Herna, I., & Hernandez, M. (n.d.). Incidental findings in imaging diagnostic tests: a systematic review. https://doi.org/10.1259/bjr/98067945

[2] Nazi, Z. A., & Peng, W. (2024). Large Language Models in Healthcare and Medical Domain: A Review. Informatics, 11(3), 57. https://doi.org/10.3390/informatics11030057

[3] https://optellum.com/

[4] https://eonhealth.com/

Summary of the Invention

In an embodiment there is provided a patient discovery system for analysing a database of clinical data containing clinical text associated with a plurality of patients; the patient discovery system comprising: a language adaptor circuit to receive a patient discovery configuration in a query language and identify the query language, wherein the patient discovery configuration is used to automatically determine one or more patients to be flagged for further clinical investigation; a data transformation circuit to receive the clinical text from the database and produce transformed clinical data for each of the plurality of patients; wherein the clinical text is in one or more source languages that are different to the query language of the patient discovery configuration; wherein the language adaptor circuit is agnostic to the one or more source languages and the query language, and receives the transformed clinical data, and detects the source language of the transformed clinical data, and uses the received transformed clinical data and the received patient discovery configuration s to produce a coupled representation; and a patient discovery circuit for receiving the coupled representation, and automatically determining from the coupled representation if the clinical text from the database of clinical data for a patient meets the requirements of the patient discovery configuration in the query language, and if the requirements of the patient discovery configuration are met the patient is added to a flag list for further investigation.

Preferably, the language adaptor circuit transforms at least one of the patient discovery configuration and the clinical data containing clinical text into one or more embedding vectors to preserve the semantics of the original text in the language adaptor circuit.

Further preferably, the coupled representation of the patient discovery configuration and the clinical text data is in one language.

In a preferred embodiment, claim further comprising an entity localiser circuit, receiving input from at least one of the language adaptor circuit and the patient discovery circuit used to localise one or more entities in the original clinical text in the source language of the original clinical text ,where the one or more entities contributed to the patient being added to the flag list. Further preferably, the entity localiser circuit comprises: a span transformation prediction model, and a span transformer; wherein a pipeline record is provided from the data transformation circuit to the span transform prediction model, and the span transform prediction model uses the pipeline record and the coupled representation to determine a span of the one or more entities in the original clinical text.

Preferably, the span transform prediction model is at least one of :a Large Language Model (LLM) using a prompt to compile steps in the pipeline record to determine the span in the original clinical text, a model using similarity of embedding vectors to match tokens in the entity with a span of tokens in the original clinical text. Further preferably, the token matching uses one or more similarity metrics to localise the span in the original clinical text.

In an embodiment, the language adaptor circuit is configured to perform at least one of: translate the query language of the patient discovery configuration to the source language of the clinical text, translate the source language of the clinical text the query language of the patient discovery configuration. Further preferably, the language adaptor circuit is configured to translate one or more of the patient discovery configuration and the clinical text into an anchor language. In a preferred embodiment at least one of the query language and the source language is a different language to the anchor language.

Preferably, the translation to an anchor language is done using a machine learning translation model. Further preferably, the machine learning translation model is a large language model configured to follow instructions from a text prompt. In an embodiment the large language model follows instructions from the text prompt to perform the steps of language adaptor, patient discovery and entity localiser.

Preferably, the patient discovery configuration is provided simultaneously in one or more different query languages.

In a preferred embodiment, the patient discovery query relates to at least one of: a specific anatomical structure, a pathological condition or a diagnostic finding.

Further preferably, the clinical text comprises one or more of the following: text based data, text information on medical images, structured text data, unstructured text data.

In an embodiment, the data transformation circuit removes any protected health information from the clinical text before providing the output to the language adaptor circuit.

Preferably, the patient discovery circuit uses a model that is at least one of: a language and text model; an embedded space representation based on mathematical operations.

In a further embodiment there is also provided a computer implemented method for analysing a database of clinical text associated with a plurality of patients; the method comprising the steps of: receiving a patient discovery configuration in the query language, wherein the patient discovery configuration is used to automatically determine one or more patients to be flagged for further clinical investigation; receiving the clinical text from the database at a data transformation circuit, and producing transformed clinical data; wherein the clinical text is in one or more source languages that are different to the query language of the patient discovery configuration; identifying the query language of the patient discovery configuration, and the source language of the clinical text; receiving the transformed clinical data and the patient discovery configuration and transforming these two received inputs into a coupled representation; receiving the coupled representation at a patient discovery circuit, and determining if the clinical text for a patient meets the requirements of the patient discovery configuration, and if the requirements are met adding the patient to a flag list for further investigation.

Preferably, the method further comprises the step of transforming at least one of the patient discovery configuration and the clinical text into one or more embedding vectors which preserve the semantics of the original text.

## Brief Description of the Figures

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.

Figure 1: illustrates a general configuration of the language agnostic patient discovery system according to an embodiment;

Figure 2: illustrates a schematic of a Data transformation circuit according to an embodiment;

Figure 3: illustrates a schematic of a Language adaptor circuit according to an embodiment;

Figure 4: illustrates a schematic of a Patient discovery circuit according to an embodiment;

Figure 5: illustrates a schematic of an Entity localiser circuit according to an embodiment;

Figure 6: illustrates a Language-agnostic patient discovery by translation of a radiology report according to an embodiment of the invention;

Figure 7: illustrates a Language-agnostic patient discovery by translation of a patient discovery configuration according to an embodiment of the invention;

Figure 8A-B: illustrates a translation model logic for translation into an anchor language according to an embodiment of the invention;

Figure 9: illustrates a Language-agnostic patient discovery by simultaneous translation of a report, and configuration into a feature vector according to an embodiment;

Figure 10: illustrates a Language-agnostic patient discovery through instruction-following large language model according to an embodiment;

Figures 11A-F: show assorted Illustrations of various steps in the processing pipeline of the language-agnostic patient discovery system according to an embodiment.

## Detailed Description

### Overview of the invention

The herein described invention relates to a language-agnostic system and method for patient discovery within healthcare information systems. More specifically, the invention provides an automated approach for flagging, from within a hospital or health care system database of patients, those relevant patients according to a relevancy criteria defined by the users of the system such as a clinic or group within the hospital. Crucially, the discovery system in this invention allows for the flagging of the patients based on clinical text data without regard to the language of such a text or the query language used to describe the relevancy criteria. For example, the invention can be used to perform the routine discovery and flagging of patients based on a relevancy criteria written in a first query language (e.g. English), which is then used to search over a database of patients containing clinical text data in one or more different languages (e.g. Spanish, French). This approach significantly reduces the time and resources required to develop and deploy patient cohort identification systems while maintaining accuracy and consistency across multiple different languages.

To achieve this, the invention is broken down into four stages: a data transformation circuit (120), language adaptor circuit (130), patient discovery circuit (160), and an entity localiser circuit (150).

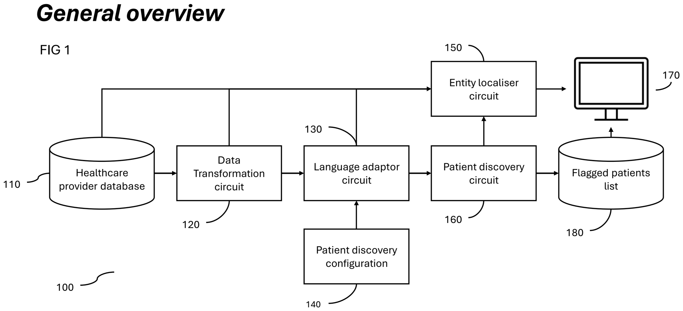

FIG. 1 shows a high-level layout of the invention (100). Firstly, clinical data containing clinical text are retrieved from a healthcare provider database (110) by the data transformation circuit (120). The data transformation circuit conducts necessary pre-processing of the retrieved data, which optionally include data de-identification and section segmentation, for instance in radiological reports. The transformed data and patient discovery configuration (140) are then passed to the language adaptor circuit (130). The patient discovery configuration is the set of parameters required by the patient discovery circuit (160) to perform the relevancy criteria-based comparison procedure, for example, specifying inclusion, exclusion and ignore criteria for a record match. In some embodiments, the parameters are regular expression based rules used to match entities of interest in a radiology report, in other embodiments the parameters are natural language queries used by a language model to determine the level of similarity between the given query, and a radiology report or subsets thereof.

The language adaptor circuit (130) takes the transformed clinical text data and patient discovery configuration (140) as input, and transforms them into a coupled representation in a shared domain. In some embodiments, the shared domain is a target language, in others the shared domain is a feature space encoded by a language encoder. The coupled representations of clinical text data (e.g. radiological report) and patient discovery configuration are passed as input to the patient discovery circuit, where the relevancy criteria-based retrieval processes takes place.

The patient discovery circuit (160) takes as input the coupled representations of the patient discovery configuration and the transformed data. The patient discovery model then determines if the given report meets the relevancy criteria. Any instance where the relevancy criteria is met is deemed to be a positive result and the patient is flagged as a result, adding the patient into a flagged patients list (180) that is made available to the users of the system (170).

Finally, the entity localiser circuit (150) is used to localise (for example, highlight) any entities of interest in the original text-based data of a patient that led to the patient being flagged. Entities of interest typically correspond to findings that may be associated with pathologies, which are reported in clinical text such as radiological reports and other types of clinical notes or patient records. The entity localiser circuit uses the outputs from other circuits in the system to determine the text span of entities of interest in the original text for use in visualisation of entities of interest on a user display (170), and other subsequent analysis steps. For example, the specific segments in the original text data that are referring to an entity of interest can be highlighted such that a user of the system can verify the validity of an particular flagged patient in the output of the language-agnostic patient discovery system.

This invention offers manufacturers of systems for text-based patient discovery a scalable way to support multiple different languages (e.g. across different regions) without the need to re-develop the product in each of the target languages. Moreover, it offers manufacturers the capability to develop the core of the system in a language where NLP tools may be widely available such as English, and still make such system available in other non-English languages without any loss of functionalities or accuracy. Likewise, the deployment of the system in this invention offers the flexibility to users of configuring the discovery system in their native language.

As described, there is a patient discovery system for analysing a database of clinical data containing clinical text associated with a plurality of patients; the patient discovery system comprising: a language adaptor circuit to receive a patient discovery configuration in a query language and identify the query language, wherein the patient discovery configuration is used to automatically determine one or more patients to be flagged for further clinical investigation; a data transformation circuit to receive the clinical text from the database and produce transformed clinical data for each of the plurality of patients; wherein the clinical text is in one or more source languages that are different to the query language of the patient discovery configuration; wherein the language adaptor circuit is agnostic to the one or more source languages and the query language, and receives the transformed clinical data, and detects the source language of the transformed clinical data, and uses the received transformed clinical data and the received patient discovery configuration s to produce a coupled representation; and a patient discovery circuit for receiving the coupled representation, and automatically determining from the coupled representation if the clinical text from the database of clinical data for a patient meets the requirements of the patient discovery configuration in the query language, and if the requirements of the patient discovery configuration are met the patient is added to a flag list for further investigation.

A computer implemented method for analysing a database of clinical text associated with a plurality of patients is also described. The method comprising the steps of: receiving a patient discovery configuration in a query language, wherein the patient discovery configuration is used to automatically determine one or more patients to be flagged for further clinical investigation; receiving the clinical text from the database at a data transformation circuit, and producing transformed clinical data; wherein the clinical text is in one or more source languages that are different to the query language of the patient discovery configuration; identifying the query language of the patient discovery configuration, and the source language of the clinical text; receiving the transformed clinical data and the patient discovery configuration and transforming these two received inputs into a coupled representation; receiving the coupled representation at a patient discovery circuit, and determining if the clinical text for a patient meets the requirements of the patient discovery configuration, and if the requirements are met adding the patient to a flag list for further investigation

The various circuits making up the language agnostic patient discovery system and method are detailed next, followed by example implementations for different use cases or technical settings.

**Data Transformation Circuit**

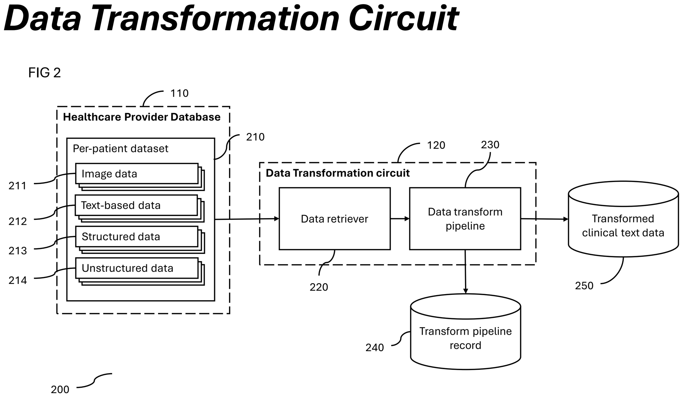

FIG. 2 shows a schematic of the data transformation circuit (200) according to an embodiment. The purpose of the data transformation circuit (120) is to perform the retrieval of clinical text data (e.g. clinical notes and reports or other text information) from the healthcare provider database (110), as well as any pre-processing steps required by subsequent circuits in a preprocessing pipeline. Typically, a healthcare provider database would store patient data in a per-patient (210) dataset. These datasets comprise modalities such as medical images, radiology reports, electronic medical records and clinical notes, and can be broadly categorised into medical image data (211), text-based data (212), structured data (213) including structured text data, and unstructured data (214), including unstructured text data.

Medical data often contains protected health information (PHI). PHI refers to individually identifiable health information that is created, received, maintained, or transmitted by healthcare providers. There are specific identifiers that constitute PHI, including names, dates, phone numbers, email addresses, and medical record numbers - all of which must be protected to maintain patient privacy. Therefore, a data anonymisation step may occur in an embodiment if required in the context of its deployment, such as being transmitted to a third-party server.

Within the data transformation circuit the data retriever (220) retrieves data from the healthcare provider database. The data transformation circuit may also responsible for refining the healthcare data, into an appropriate condition for downstream processing by subsequent parts of the pipeline (230). Refinement of the data may include steps such as formatting, filtering, redaction, or the computation of additional variables, for instance, derived from the existing data. A record of the transformation steps carried out during preprocessing are then passed to the transform pipeline record (240), where it can be used downstream by entity localiser circuit (500).

The output (250) of the data transformation circuit (120) is a transformed set of data according to the operations configured in the dataset transformation pipeline (230).

**Language Adaptor Circuit**

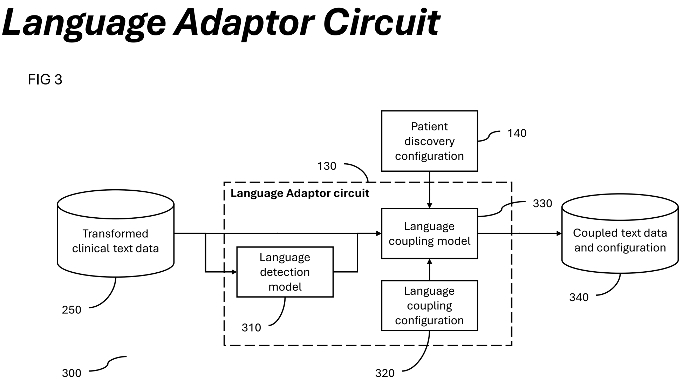

FIG. 3 shows an example configuration (300) of the language adaptor circuit (130), where it receives the transformed data (250) from the data transformation circuit (120) as input, and additionally the patient discovery configuration (140). The language adaptor circuit (130) executes the process which couples the patient discovery configuration (140) and the transformed data (250) into representations in compatible domains. Preferably, the language adaptor circuit is configured to receive the patient discovery query in the query language, and to receive the transformed clinical text data and to transform these two received inputs into a coupled representation. Further preferably, the language adaptor circuit identifies the language of the patient discovery query and detects the language of the transformed patient clinical text data. In a preferred embodiment, the coupled representation is in a single language. Further preferably in the system and the method as described the language adaptor circuit transforms at least one of the patient discovery query and the clinical text into one or more embedding vectors which preserve the semantics of the original text.

The example configuration (300) of the language adaptor circuit (130) comprises a language detection model (310) that identifies the language contained in the clinical text data, a language coupling configuration (320) containing the parameters required by the language coupling model, and the language coupling model (330). The language detection model (310) is an automated method used to determine the identity of a language of the clinical text from a sample of text in that language. In some embodiments, this is achieved using a machine learning (ML) model, dictionary-based methods, or character set analysis. The language coupling model (330) is a model capable of translating a text string from one language to another. Using for example, rule-based methods, statistical-based methods, or ML based methods. The language coupling configuration (320) contains the parameters required by the language coupling model (330), such as the source and target language of the translation task. Preferably, the language adaptor circuit is configured to translate one or more of the patient discovery query and the clinical text into an anchor language. In a preferred embodiment at least one of the query language and the source language is a different language to the anchor language. In a preferred embodiment, the translation to an anchor language is done using a machine learning translation model. Further preferably, the machine learning translation model is a large language model configured to follow instructions from a text prompt. In an embodiment, the large language model follows instructions from the text prompt to perform the steps of language adaptor, patient discovery and entity localiser

The language coupling configuration (320) is used in conjunction with the detected language of the clinical text from the database (110), to form the full configuration set for the language coupling model (330). The language coupling model performs the coupling step and passes the resulting coupled patient discovery configuration and text data (340) to the patient discovery circuit for further processing.

In some examples of the language adaptor circuit (130), the language coupling model (330) operates in an embedding space (e.g. feature vectors), thus transforming either clinical text data (250), the patient discovery configuration (140) or both, into mathematical representations such as vectors that preserve the semantic meaning of the original text, where the language coupling configuration (320) define the parameters of the embedding space representation. In this example, the coupled text data and configuration (340) output by the language adaptor circuit contains the embedding space representations generated by the language coupling model (330).

**Patient discovery circuit**

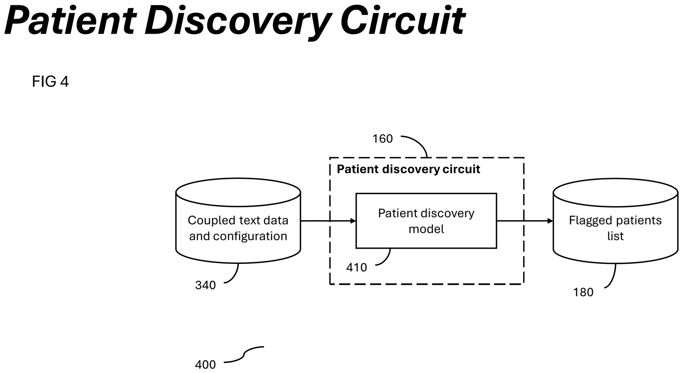

FIG. 4 shows an example schematic (400) of the patient discovery circuit (160). The patient discovery circuit (160) contains a patient discovery model (410), which performs the process of flagging patients into a list of flagged patients (180), according to information in the clinical text from the patient database. The patient discovery model receives the coupled text data and configuration (430) from the language adaptor circuit (130), and outputs a binary variable indicating whether the patient meets the criteria given by the patient discovery configuration (140), adding those flagged patients into a flagged patient list (180) made available to the users of the system (170) or more generally, to any hospital IT system that is configured to make use of the patient list.

Like the language adaptor circuit (130), in some examples of the invention, the patient discovery circuit (160) operates based on coupled text data and configuration (340) existing in a human language, while in other examples, it operates in an embedding space representation such as feature vectors. In those implementations where the coupled text data and configuration (340) consist of a human language (e.g. English text), the patient discovery model (410) performs the process of determining whether the criteria given by the patient discovery configuration (140) matches the text data corresponding to a patient by using language-based techniques such as regular expression pattern search or term frequency-inverse document frequency (TF-IDF). In those implementations where the coupled text data and configuration (340) consist of embedding representations, the patient discovery model (410) is based on arrangements of mathematical operations. In some examples, these mathematical operations are similarity metrics such as cosine or L2 similarities. In other examples, the mathematical operations are statistical matches such as implemented as machine learning models such as Large Language Models (LLM). In a preferred embodiment, the patient discovery circuit uses a model that is at least one of: a language and text model; an embedded space representation based on mathematical operations.

**Entity localiser circuit**

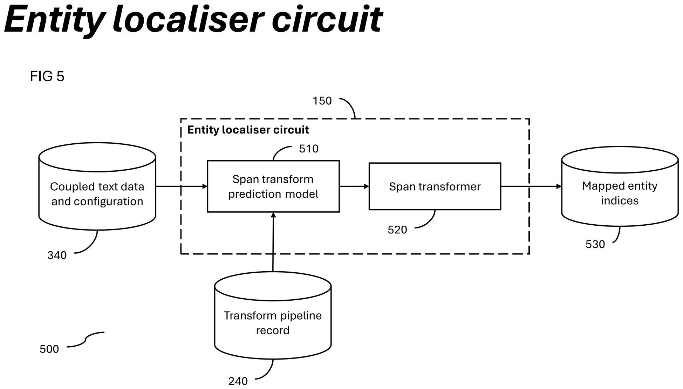

An example configuration (500) of the entity localiser circuit (150) is shown in FIG. 5. The entity localiser circuit (150) provides a mechanism by which text spans of interest detected by the patient discovery circuit (160), are localised in the original clinical text data in a patient dataset (210). A text span is considered of interest if it contains an matched entity $\lambda$, that is, an entity mentioned in the clinical text which matches the patient discovery query. The entity localiser circuit (150) consists of a span transform prediction model (510), and span transformer (520). Preferably, the entity localiser circuit is used to localise one or more entities in the original clinical text in the original source language, that contributed to the patient being added to the flagged patient list (180).

The span transform prediction model (510) determines how to map identified entities from the coupled text data and configuration (340) back to their original location in the clinical text data of a patient dataset (210). The span transform prediction model (510) determines the span of the entity in the original clinical text data (210) by taking the transform pipeline record (240) and the coupled text data and configuration (340) as input and performs a prediction to determine the span in the original text. In some examples, this is done by translating the span of the entity in the coupled text data into the language of the original clinical text data (210) and looking for occurrences of the translated text by matching regular expression patterns. In some embodiments of this invention, the span transform prediction model (510) may be an LLM, which uses a prompt configured to compile each step in the transform pipeline record (240) together, and implicitly determine the corresponding span in the original clinical text data. In other embodiments, the span transform prediction model (510) uses the similarity of word embeddings to match the tokens contained within the matched entity span, to a span of tokens in the original clinical text data of a patient dataset (210). For example, using a language encoder $\varepsilon$ capable of converting text into vectorised representations, the input text is tokenised into n sub-strings $w_1, \dots, w_n$ and converted to $n$ vectors $v_{text,1}, \cdots, v_{text,n}$. In some examples of this embodiment, $\varepsilon$ is a word embedding model such as Word2Vec, in other examples of this embodiment $\varepsilon$ is an encoder LLM such as the Bidirectional Encoder Representation from Transformers (BERT)

$$v_i^{text} \;=\; \mathcal{E}(w_i) \in \mathbb{R}^d, i \;=\; 1, \dots, n$$

Similarly, the matched entity $\lambda$ is vectorised using $\varepsilon$:

$$v^\lambda \;=\; \mathcal{E}(\lambda) \in \mathbb{R}^d$$

In cases where the matched entity consists of multiple tokens, each token is embedded separately. An aggregate function is used to reduce the vector set to a singular vector. For example, where an entity consists of k tokens, the set can be reduced using an arithmetic mean.

$$v_j^\lambda \;=\; \mathcal{E}\left(\lambda_j\right) \in \mathbb{R}^d, j = 1, \dots, k$$

$$v^\lambda \;=\; \frac{\sum_{j=0}^{k} v_j^\lambda}{k}$$

To localise the entity of interest within the text report, the similarity of each word embedding in the input text $v_i^{text}$, and the matched entity embedding $v^\lambda$ are computed using an appropriate similarity metric, for instance L2 distance:

$$L2_i = |v_i^{text} - v^\lambda|_2 = \sqrt{\sum_{j=1}^{d} \left(v_{text,i,j} - v_{\lambda,j}\right)^2}$$

Or cosine similarity ($\Theta$):

$$\Theta_i = \frac{v_i^{text} \cdot v^\lambda}{|v_i^{text}||v^\lambda|} = \frac{\sum_{j=1}^{d} v_{i_j}^{text} v_j^\lambda}{\sqrt{\sum_{j=1}^{d}\left(v_{i_j}^{text}\right)^2}\sqrt{\sum_{j=1}^{d}\left(v_j^\lambda\right)^2}}$$

Once the similarity of each word in the input text has been determined, an appropriate threshold can then be applied to localise the entity discretely.

**Embodiments of the language-agnostic patient discovery system**

Various example embodiments of the language-agnostic patient discovery system are presented next. These address different use cases or configurations of this invention, and detail how individual circuits of the system are configured accordingly.

*Language-agnostic patient discovery by translation of radiology reports.*

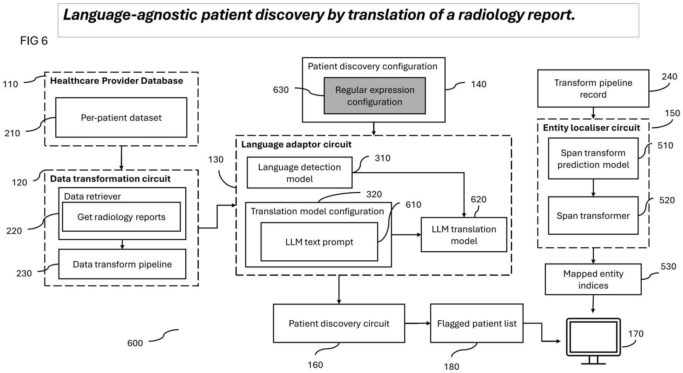

An embodiment of the invention is represented in FIG.6, where the input per-patient dataset (210) consisting of a clinical text data comprising a radiology report is translated from the original source language before undergoing the patient discovery process (600). In this example embodiment (600) of the language-agnostic patient discovery system, a radiology report is retrieved from the healthcare provider database (110), translated from the source language to a target language, and used in conjunction with a set of regular expression based rules to perform the patient discovery step. In this example, the target language for translation is the language of the query in the patient discovery configuration (140) such as a query in the form of a regular expression pattern (630). The regular expression pattern (630) is used to specify a set of rules, enabling the patient discovery model to detect a span of interest within a report, such as a specific anatomical structure, pathological condition, or diagnostic finding that can identified in the text of a radiology report.

The radiology report is retrieved from the healthcare provider database by the data retriever (220) within the data transformation circuit (120). The report is then passed to the data transformation pipeline (230) for pre-processing, for instance, where any PHI present in the report is redacted, and the patient history and technique section are removed. After pre-processing, the data is passed to the language detection module (310) within the language adaptor circuit (130), which predicts the language of the report. The predicted language and report are inserted into a template to form the LLM text prompt (610), which is then passed to an LLM suited for language translation. In some example embodiments, the LLM prompt template is:

*"Translate the text below from {{SOURCE LANGUAGE}} to {{TARGET LANGUAGE}}. The response should contain only the translated text. TEXT: {{CLINICAL TEXT REPORT}}"*

In the prompt template example, variables defined within double curly brackets are substituted during the language translation process. The LLM outputs the translated report, which is then passed to the patient discovery circuit (160).

The translated report is passed as input to the patient discovery model (410), within the patient discovery circuit (160). In an example of this embodiment, the patient discovery model (410) is used with the patient discovery configuration (140), consisting of a regular expression pattern (630) designed to assert the presence of an entity of interest. For example, the following regular expression pattern is configured to find reports or clinical text data containing mentions of lung nodules or masses:

*Configuration = "(lung|pulmonary)\s+(nodules?|mass(es)?)"*

If the report or clinical text data contains at least one match of the query defined by the regular expression pattern given in the configuration (140), the output of the model is positive and the patient is added to the flagged list of patients (180), otherwise, if it is negative, and there is no match, the patient is not added to the flagged list.

The outputs of the data transformation (120), and language adaptor circuits (130) in an example of this embodiment are also passed to the entity localiser circuit (150) through a transform pipeline record (240). The output of each step is then collated, and used by the span transform prediction model (510) to determine the indices of any entities of interest determined by the patient discovery model (410). The results of the entity localiser circuit (150) may then be used to visualise any occurrences of any terms in the patient discovery configuration (140) that lead to a patient to be added to list of flagged patients (180). For instance, for the example configuration of lung nodules or masses presented above, the mapped entity indices (530) produced by the entity localiser circuit (150) allows the highlighting of mentions of lung nodules or masses in the original text data (210) corresponding to a flagged patient, regardless of the language in which that text was written.

*Language-agnostic patient discovery by translation of a patient discovery configuration .*

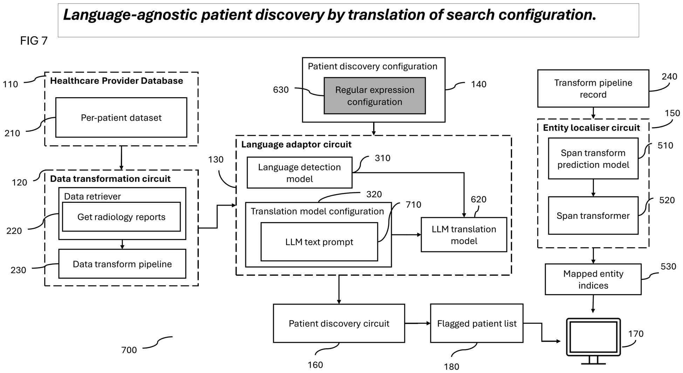

In this example embodiment of the language-agnostic patient discovery system, the patient discovery configuration (140) is translated from the original query language by the language adaptor circuit in order to match the source language of the clinical text data in the patient datasets (210). In some examples of this embodiment where the patient discovery configuration (140) is a regular expression pattern, an LLM translation model is used to translate the regular expression pattern, designed to assert the presence of an entity of interest, into the language of the report or clinical text data contained within the healthcare database (110). An overview of this embodiment is shown in FIG. 7.

Firstly, a text-based report or other clinical text data is retrieved from the healthcare provider database (110) by the data retriever (220) of the data transformation circuit. It is then passed to the data transform pipeline (230) within the data transformation circuit (120) where pre-processing takes place, and then to the language detection module (310) within the language adaptor circuit (130) to determine the original source language of the clinical text data. In an example of this embodiment, the original source language of the report is determined using an LLM, which has been configured to detect the language of a sample of input text. The name of the detected source language is inserted into a pre-defined LLM prompt template (710), which is then provided to an LLM translation model (620). The prompt template instructs the LLM to translate the regular expression patterns (630) into the detected language of the clinical text data, for instance, through the following example prompt.

*"Translate the regular expression pattern stated after PATTERN: from {{SOURCE LANGUAGE}} into {{TARGET LANGUAGE}}. Ensuring that any terms and look around functionality is maintained. Respond only with the converted regular expression pattern.*

*PATTERN: {{REGULAR EXPRESSION}}"*

The translated regular expression patterns and transformed clinical text data can now be used by the patient discovery model (410) to determine whether the patient should be flagged into the flagged patients list (180) according to the (now translated) relevancy criteria in the patient discovery configuration (140). Finally, the entity localiser circuit (150) by having access to the record of data transformations in the transformation pipeline record (240), can estimate which text spans in the original patient dataset (210) correspond to those requested in the patient discovery configuration (140) such that they can be highlighted when a user is reviewing the list of flagged patients (180) in a user interface (170).

*Language-agnostic patient discovery by simultaneous translation of reports, and configuration into an anchor language.*

In a further example embodiment, the clinical text in the per-patient dataset (210) and patient discovery configuration (140) can be both translated into a preconfigured reference language as required - referred to henceforth as an *anchor language.* In an example of this embodiment, the clinical text or report is in French, the patient discovery configuration (140) is in Spanish, and the anchor language is English. Thus, it is preferable to translate the clinical text or report from French to English, and to translate the patient discovery configuration from Spanish to English. In a preferred embodiment the patient discovery query is provided simultaneously in one or more different query languages.

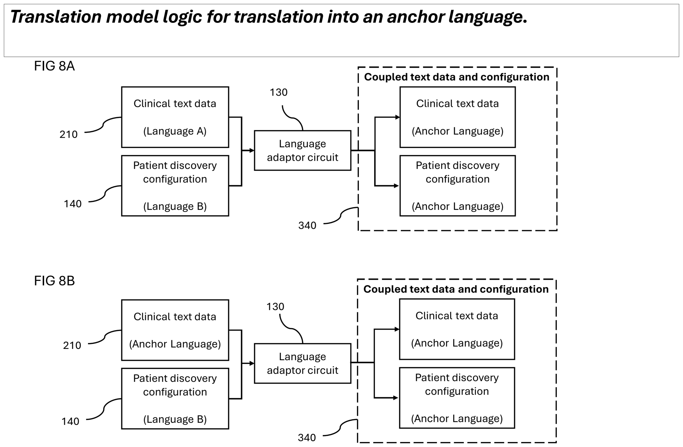

This example embodiment proceeds similarly to previous embodiments where either the patient clinical text data (210) or the report, or patient discovery configuration (140) were translated. However, the language adaptor circuit (130) now makes the determination of which pieces of text required translation and executes it accordingly. For instance, FIG. 8A presents a scenario where both the clinical text data or report in a patient dataset (210) and the patient discovery configuration are both in languages that are different from the anchor language. In this scenario, both units of data, that is the clinical text or report from the patient dataset, and the patient discovery configuration are translated into the anchor language resulting in the coupled text data and configuration (340) required by the patient discovery circuit.

FIG. 8B presents a scenario where the clinical text data (210) or report is already in the anchor language, thus requires no translation; only translating the patient discovery configuration (140) into the anchor language from the original query language is required to obtain the coupled text data and configuration (340). The operation of the entity localiser circuit (150) is preserved from that described in the previous embodiments.

*Language-agnostic patient discovery by simultaneous translation of reports, and configuration into a feature vector.*

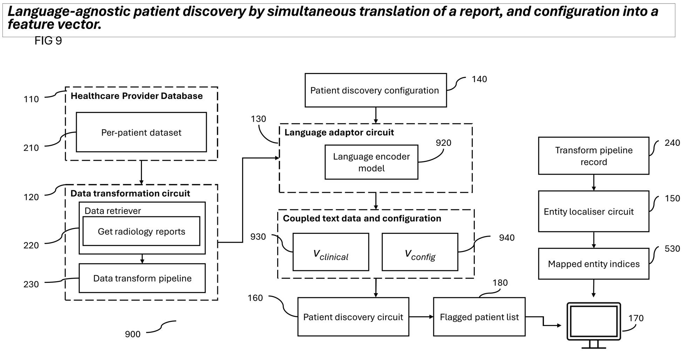

In an example embodiment (900) depicted in FIG. 9, a clinical text report within a patient dataset (210) and a patient discovery configuration (140) are mapped to feature vector embeddings using a language encoder model (920), resulting in encodings denoted as $v_{clinical}$ (930), for the clinical text data or report, and $v_{confg}$ (940), for the patient discovery configuration. The patient discovery configuration (140) in this example of the embodiment is a natural language query designed to detect mentions of specific keywords in the report, in this example the keywords relate to either a nodule or mass in the report text:

*Natural language query:*

*"Identify mentions of any pulmonary nodules or masses"*

The patient discovery configuration (140) may contain any valid representation of an entity that can be mapped to a feature vector embedding using a language encoder model. These feature embedding vectors $v_{clinical}$ (930) and $v_{config}$ (940) capture the contextualised semantic information of their respective text, and are used to search whether the semantics of $v_{config}$ (940) occur in $v_{clinical}$ (930). Such a comparison is performed with mathematical operations such as vector similarity metrics.

As in previous embodiments, the report or clinical text data within a patient dataset (210) is sourced from the healthcare provider database (110) and passed to the data transformation circuit (120) for retrieval (220) and preprocessing (230). The patient discovery configuration (140) and processed clinical text data are passed to the language adaptor circuit (130) which implements a language encoder model (920). The language encoder model $\varepsilon$, converts the processed clinical text data r to its embedding vector $v_{clinical}$ (930) of dimensionality d.

$$v_{clinical} = \mathcal{E}(r) \in \mathbb{R}^d$$

Similarly, the patient discovery configuration c (140) is mapped to an embedding $v_{config}$ (940):

$$v_{config} = \mathcal{E}(c) \in \mathbb{R}^d$$

In some examples of this embodiment, the similarity of the clinical text data $v_{clinical}$, and the configuration $v_{config}$ are computed using cosine similarity:

$$\text{Cosine Similarity} = \frac{v_{clinical} \cdot v_{config}}{|v_{clinical}| \, |v_{config}|} = \frac{\sum_{j=1}^d v_j^{clinical} v_j^{config}}{\sqrt{\sum_{j=1}^d \left(v_j^{clinical}\right)^2} \sqrt{\sum_{j=1}^d \left(v_j^{config}\right)^2}}$$

To determine the relevancy of $v_{config}$ in the context of $v_{clinical}$, a threshold is applied to the computed similarity metric value by the patient discovery model (410) within the patient discovery circuit (160). A report with a similarity above this threshold is deemed to be relevant. If the report belonging to a patient is deemed to be relevant, the patient is added to flagged patient list (180).

Finally, the entity localiser circuit (150) can use the transform pipeline record (240) to determine the entities in the original clinical text data (210) that should be highlighted when inspecting the matched entities on a flagged patient. Specifically, the entity localiser circuit (150) can inspect which feature embedding vectors in $v_{clinical}$ returned a high similarity or match to $v_{config}$, while also having access to which strings in the original clinical text data where used to produced such matching feature embedding vectors. As a result, the mapped entity indices (530) at the output of the entity localiser circuit simply index the position of those identify strings.

*Language-agnostic patient discovery using a unified language coupling and patient discovery model.*

In a further example embodiment (1000), an instruction-following large language model (LLM) capable of translation and entity identification based on a pre-defined set of criteria is used to perform multiple steps of this invention internally, thus englobing the tasks of the language adaptor circuit (130), the patient discovery circuit (140) and the entity localiser circuit (150). Such an LLM is typically trained as a general language model through proxy tasks such as self-supervised text completion from multi-lingual text, and is then fine-tuned to follow instructions through techniques such as Reinforcement Learning with Human Feedback (RLHF) or Direct Preference Optimization (DPO).

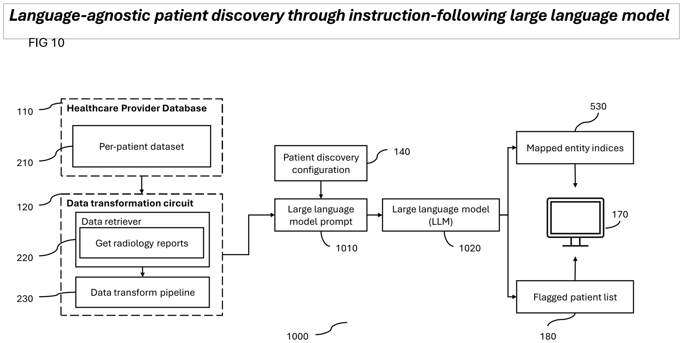

A schematic of this embodiment is shown in FIG. 10. Text-based reports or other clinical data are stored in a healthcare provider database (110) and are retrieved (220) and pre-processed (230) by the data transformation circuit (120). The processed reports or clinical text and the patient discovery configuration (140) are then built into a text-based prompt (1010) for the LLM. For example, the patient discovery configuration (140) consists of a natural language query, written in English, designed to determine the existence of pulmonary nodules in Spanish :

*"Find any mention of pulmonary nodules in this radiology report, output 'true' if there is at least one instance, otherwise*

*output 'false'*

*{CLINICAL TEXT REPORT (Spanish)}"*

The LLM prompt (1010) now contains all the required information to perform the language adaptor and discovery tasks, feeding its output directly to the flagged patient list.

Finally, the instruction-following LLM can also be prompted to perform the task of the entity localisation circuit (150) by supplementing the prompt template (1010):

*"Output the text spans in the clinical text report that mention the entities being searched for"*

Such an output is then used to generate the respective indices (530) on the clinical text data that are required to highlight the matched entities for the flagged patients.

**Illustration of steps within language agnostic patient discovery system.**

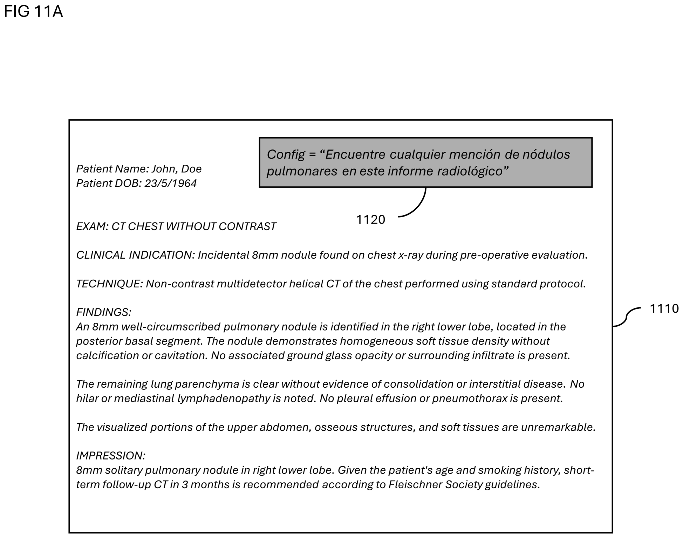

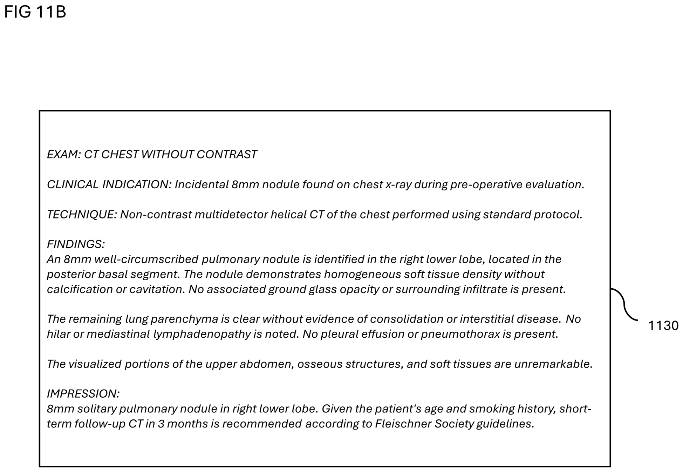

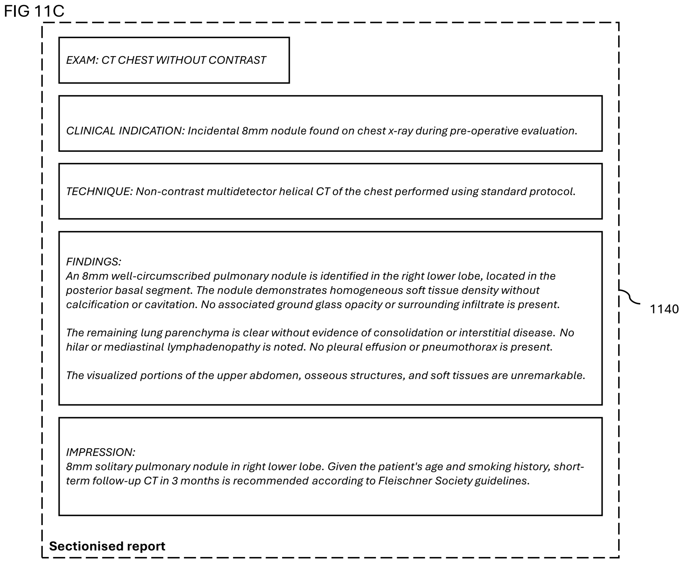

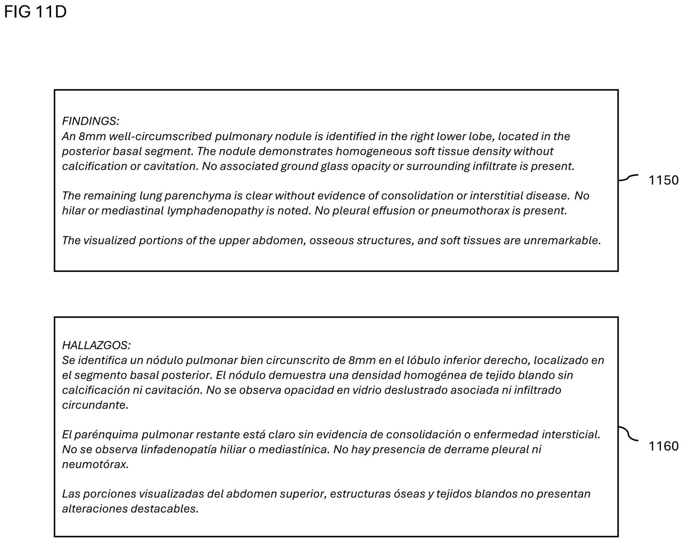

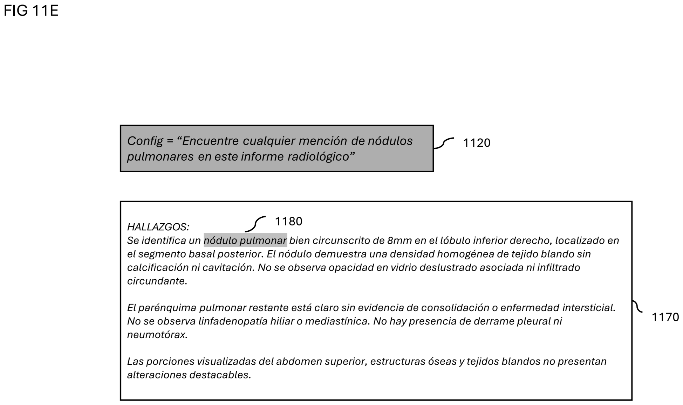

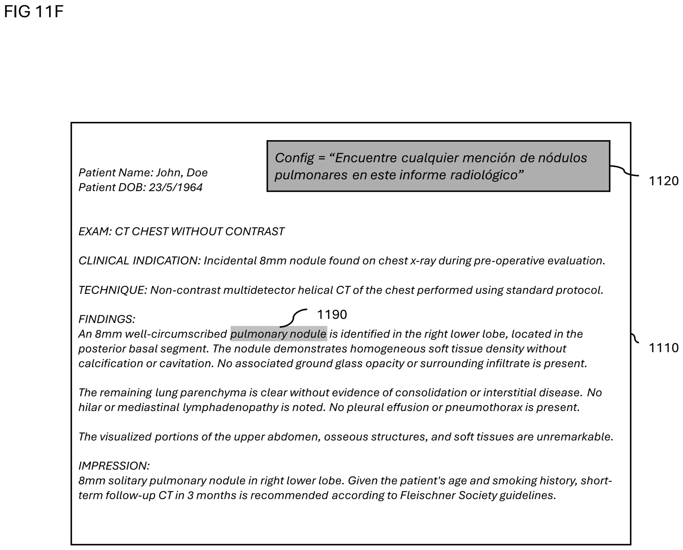

The sequence of steps in an exemplar implementation of the invention are represented in FIG. 11A-F. FIG. 11A shows the inputs required by the specific implementation, consisting of a raw radiology report in English (1110) and a Spanish natural language query as the configuration (1120) - designed to detect any mention of a pulmonary nodule in the radiology report. FIG. 11B shows the report after having undergone anonymisation (1130) to remove any potential PHI from the report. FIG. 11C shows the radiology report after paragraph-based segmentation (1140). FIG. 11D shows the findings section of the radiology report before (1150) and after (1160) translation by the language adaptor circuit (130). FIG. 11E shows the output of the patient discovery circuit (160). The translated findings section of the report (1170) is shown with a highlighted mention of a pulmonary nodule (1180) as described in the Spanish natural language query (1120). The form of the report which would typically be visualised by the end user is shown in FIG. 11F. The natural language configuration is shown (1120) with the report in its original form (1110) annotated (1190) by any desired entities described in the configuration.

This implementation demonstrates the complete workflow from input to output: starting with a raw English radiology report and Spanish natural language query, proceeding through anonymisation, segmentation, and translation steps, and culminating in an annotated report highlighting the requested clinical findings.

In an embodiment there is provided a language-agnostic patient discovery system for flagging, from within a database of clinical text data or reports associated with patients, those relevant patients according to a patient discovery configuration criteria in a query language for the clinical text data, wherein a language adaptor circuit couples the query language of the discovery configuration and the language of the clinical text data or report such that the system is language agnostic. The system may use one or more different languages simultaneously for a patient discovery query, as well as different languages for the clinical text data or reports.

Preferably when relevant text spans in the clinical text data or report match the patient discovery criteria leading to the flagging of a patient, the relevant text spans can be can be highlighted in the clinical text data in its original source language, that may be different to the query language of the patient discovery criteria..

In a preferred embodiment, the language adaptor circuit is configured to translate the patient discovery configuration from the query language of the patient discovery configuration into the source language of the clinical text data.

In a further preferred embodiment the language adaptor circuit is configured to translate the clinical text data or the report from the original source into the query language of the patient discovery configuration.

Preferably, the language adaptor circuit is configured to translate the clinical text data from the source language and patient discovery configuration in the query language, into an anchor language. Preferably, the translation of at least one of the source language and the query language into an anchor language is done by a machine learning translation model.

Further preferably, the translation model is a large language model configured to follow instructions from a text prompt.

In an embodiment, inputs from the clinical text data or reports are de-identified, with the removal of any personal health information (PHI) before the clinical text or reports are given to the translation model.

In a preferred embodiment, the patient discovery configuration in the query language is configurable according to exclusions, inclusions and ignore statements.

Preferably, the patient discovery configuration in the query language describes one or more clinical entities of interest, such as anatomies, pathologies, demographics, procedures and clinical interventions.

Preferably, the patient discovery configuration is agnostic to the query language (e.g. the query can be written in multiple different query languages simultaneously).

As discussed an embodiment of the patient discovery system offers manufacturers of systems for text-based patient discovery a scalable way to support multiple different languages (e.g. across different regions) without the need to re-develop the product in each of the target languages. Moreover, it offers them the capability to develop the core of the system in a language where NLP tools may be widely available such as English, and still make such a system available in other languages without the loss of functionalities or accuracy. Likewise, the deployment of the system in this invention offers the flexibility to users of configuring the discovery system in their native language.

This invention can be used in the following in assorted healthcare systems, Clinical trials and by Clinical research organizations amongst others.

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for automated contouring of cone-beam CT images.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected,' or 'operably coupled,' to each other to achieve the desired functionality.

Furthermore, those skilled in the art will recognize that boundaries between the above-described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

**Claims**

1. A patient discovery system for analysing a database of clinical data containing clinical text associated with a plurality of patients;
the patient discovery system comprising:

   a language adaptor circuit to receive a patient discovery configuration in a query language and identify the query language, wherein the patient discovery configuration is used to automatically determine one or more patients to be flagged for further clinical investigation;
   a data transformation circuit to receive the clinical text from the database and produce transformed clinical data for each of the plurality of patients; wherein the clinical text is in one or more source languages that are different to the query language of the patient discovery configuration;
   wherein the language adaptor circuit is agnostic to the one or more source languages and the query language, and receives the transformed clinical data, and detects the source language of the transformed clinical data, and uses the received transformed clinical data and the received patient discovery configuration s to produce a coupled representation; and
   a patient discovery circuit for receiving the coupled representation, and automatically determining from the coupled representation if the clinical text from the database of clinical data for a patient meets requirements of the patient discovery configuration in the query language, and if the requirements of the patient discovery configuration are met the patient is added to a flag list for further investigation.

2. The patient discovery system as claimed in claim 1 wherein the language adaptor circuit transforms at least one of the patient discovery configuration and the clinical data containing clinical text into one or more embedding vectors which preserve semantics of the original clinical text in the language adaptor circuit.

3. The patient discovery system claimed in claim 1 or 2 wherein the coupled representation of the patient discovery configuration and the clinical text is in one language.

4. The patient discovery system as claimed in any preceding claim further comprising an entity localiser circuit, receiving input from at least one of the language adaptor circuit and the patient discovery circuit, and used to localise one or more entities in the original clinical text in the source language of the original clinical text, where the one or more localised entities contributed to the patient being added to the flag list.

5. The patient discovery system as claimed in claim 4 wherein the entity localiser circuit comprises: a span transform prediction model, and a span transformer; wherein a pipeline record is provided from the data transformation circuit to the span transform prediction model, and the span transform prediction model uses the pipeline record and the coupled representation to determine a span of the one or more entities in the original clinical text.

6. The patient discovery system as claimed in claim 5 wherein the span transform prediction model is at least one of: a Large Language Model (LLM) using a prompt to compile steps in the pipeline record to determine the span in the original clinical text, a model using similarity of embedding vectors to match tokens in the entity with a span of tokens in the original clinical text.

7. The patient discovery system as claimed in any preceding claim wherein the language adaptor circuit is configured to perform at least one of: translate the query language of the patient discovery configuration to the source language of the clinical text, translate the source language of the clinical text to query language of the patient discovery configuration.

8. A patient discovery system as claimed in any preceding claim wherein the language adaptor circuit is configured to translate one or more of the patient discovery configuration and the clinical text into an anchor language.

9.  The patient discovery system claimed in claim 8 wherein at least one of the query language and the source language is a different language to the anchor language, wherein the translation to the anchor language is done using a machine learning translation model.

10. The patient discovery system claimed in claim 9 wherein the machine learning translation model is a large language model configured to follow instructions from a text prompt, to perform the steps of language adaptor, patient discovery and entity localiser.

11. The patient discovery system as claimed in any preceding claim wherein the patient discovery configuration is provided simultaneously in one or more different query languages.

12. The patient discovery system as claimed in any preceding claim wherein the patient discovery configuration relates to at least one of: a specific anatomical structure, a pathological condition or a diagnostic finding

13. The patient discovery system claimed in any preceding claim wherein the clinical text comprises one or more of the following: text based data, text information on medical images, structured text data, unstructured text data.

14. The patient discovery system as claimed in any preceding claim wherein the patient discovery circuit uses a model that is at least one of: a language and text model; an embedded space representation based on mathematical operations.

15. A computer implemented method for analysing a database of clinical text associated with a plurality of patients; the method comprising the steps of:

    receiving a patient discovery configuration in a query language, wherein the patient discovery configuration is used to automatically determine one or more patients to be flagged for further clinical investigation;
    receiving the clinical text from the database at a data transformation circuit, and producing transformed clinical data; wherein the clinical text is in one or more source languages that are different to the query language of the patient discovery configuration;
    identifying the query language of the patient discovery configuration, and the source language of the clinical text;
    receiving the transformed clinical data and the patient discovery configuration and transforming these two received inputs into a coupled representation;
    receiving the coupled representation at a patient discovery circuit, and determining if the clinical text for a patient meets requirements of the patient discovery configuration, and if the requirements are met adding the patient to a flag list for further investigation.

*General overview*

FIG 1

# *Data Transformation Circuit*

FIG 2

Healthcare Provider Database — 110

Per-patient dataset — 210

- Image data — 211
- Text-based data — 212
- Structured data — 213
- Unstructured data — 214

Data Transformation circuit — 120

Data retriever — 220

Data transform pipeline — 230

Transformed clinical text data — 250

Transform pipeline record — 240

200

# *Language Adaptor Circuit*

FIG 3

# *Patient Discovery Circuit*

FIG 4

# *Entity localiser circuit*

FIG 5

**Language-agnostic patient discovery by translation of a radiology report.**

FIG 6

**Language-agnostic patient discovery by translation of search configuration.**

FIG 7

110 Healthcare Provider Database

210 Per-patient dataset

120 Data transformation circuit

Data retriever

220 Get radiology reports

230 Data transform pipeline

Patient discovery configuration

630 Regular expression configuration — 140

130 Language adaptor circuit

Language detection model — 310

Translation model configuration — 710

LLM text prompt — 320

620 LLM translation model

Transform pipeline record — 240

150 Entity localiser circuit

Span transform prediction model — 510

Span transformer — 520

Mapped entity indices — 530

Patient discovery circuit — 160

Flagged patient list — 180

170

700

**Translation model logic for translation into an anchor language.**

FIG 8A

FIG 8B

*Language-agnostic patient discovery by simultaneous translation of a report, and configuration into a feature vector.*

FIG 9

**Language-agnostic patient discovery through instruction-following large language model**

FIG 10

FIG 11A

Config = *"Encuentre cualquier mención de nódulos pulmonares en este informe radiológico"*

1120

*Patient Name: John, Doe*
*Patient DOB: 23/5/1964*

*EXAM: CT CHEST WITHOUT CONTRAST*

*CLINICAL INDICATION: Incidental 8mm nodule found on chest x-ray during pre-operative evaluation.*

*TECHNIQUE: Non-contrast multidetector helical CT of the chest performed using standard protocol.*

*FINDINGS:*
*An 8mm well-circumscribed pulmonary nodule is identified in the right lower lobe, located in the posterior basal segment. The nodule demonstrates homogeneous soft tissue density without calcification or cavitation. No associated ground glass opacity or surrounding infiltrate is present.*

*The remaining lung parenchyma is clear without evidence of consolidation or interstitial disease. No hilar or mediastinal lymphadenopathy is noted. No pleural effusion or pneumothorax is present.*

*The visualized portions of the upper abdomen, osseous structures, and soft tissues are unremarkable.*

*IMPRESSION:*
*8mm solitary pulmonary nodule in right lower lobe. Given the patient's age and smoking history, short-term follow-up CT in 3 months is recommended according to Fleischner Society guidelines.*

1110

FIG 11B

*EXAM: CT CHEST WITHOUT CONTRAST*

*CLINICAL INDICATION: Incidental 8mm nodule found on chest x-ray during pre-operative evaluation.*

*TECHNIQUE: Non-contrast multidetector helical CT of the chest performed using standard protocol.*

*FINDINGS:*
*An 8mm well-circumscribed pulmonary nodule is identified in the right lower lobe, located in the posterior basal segment. The nodule demonstrates homogeneous soft tissue density without calcification or cavitation. No associated ground glass opacity or surrounding infiltrate is present.*

*The remaining lung parenchyma is clear without evidence of consolidation or interstitial disease. No hilar or mediastinal lymphadenopathy is noted. No pleural effusion or pneumothorax is present.*

*The visualized portions of the upper abdomen, osseous structures, and soft tissues are unremarkable.*

*IMPRESSION:*
*8mm solitary pulmonary nodule in right lower lobe. Given the patient's age and smoking history, short-term follow-up CT in 3 months is recommended according to Fleischner Society guidelines.*

1130

FIG 11C

EXAM: CT CHEST WITHOUT CONTRAST

CLINICAL INDICATION: Incidental 8mm nodule found on chest x-ray during pre-operative evaluation.

TECHNIQUE: Non-contrast multidetector helical CT of the chest performed using standard protocol.

FINDINGS:
An 8mm well-circumscribed pulmonary nodule is identified in the right lower lobe, located in the posterior basal segment. The nodule demonstrates homogeneous soft tissue density without calcification or cavitation. No associated ground glass opacity or surrounding infiltrate is present.

The remaining lung parenchyma is clear without evidence of consolidation or interstitial disease. No hilar or mediastinal lymphadenopathy is noted. No pleural effusion or pneumothorax is present.

The visualized portions of the upper abdomen, osseous structures, and soft tissues are unremarkable.

IMPRESSION:
8mm solitary pulmonary nodule in right lower lobe. Given the patient's age and smoking history, short-term follow-up CT in 3 months is recommended according to Fleischner Society guidelines.

**Sectionised report**

1140

FIG 11D

FINDINGS:
*An 8mm well-circumscribed pulmonary nodule is identified in the right lower lobe, located in the posterior basal segment. The nodule demonstrates homogeneous soft tissue density without calcification or cavitation. No associated ground glass opacity or surrounding infiltrate is present.*

*The remaining lung parenchyma is clear without evidence of consolidation or interstitial disease. No hilar or mediastinal lymphadenopathy is noted. No pleural effusion or pneumothorax is present.*

*The visualized portions of the upper abdomen, osseous structures, and soft tissues are unremarkable.*

1150

HALLAZGOS:
*Se identifica un nódulo pulmonar bien circunscrito de 8mm en el lóbulo inferior derecho, localizado en el segmento basal posterior. El nódulo demuestra una densidad homogénea de tejido blando sin calcificación ni cavitación. No se observa opacidad en vidrio deslustrado asociada ni infiltrado circundante.*

*El parénquima pulmonar restante está claro sin evidencia de consolidación o enfermedad intersticial. No se observa linfadenopatía hiliar o mediastínica. No hay presencia de derrame pleural ni neumotórax.*

*Las porciones visualizadas del abdomen superior, estructuras óseas y tejidos blandos no presentan alteraciones destacables.*

1160

FIG 11E

Config = *"Encuentre cualquier mención de nódulos pulmonares en este informe radiológico"*    1120

*HALLAZGOS:*    1180
*Se identifica un nódulo pulmonar bien circunscrito de 8mm en el lóbulo inferior derecho, localizado en el segmento basal posterior. El nódulo demuestra una densidad homogénea de tejido blando sin calcificación ni cavitación. No se observa opacidad en vidrio deslustrado asociada ni infiltrado circundante.*

*El parénquima pulmonar restante está claro sin evidencia de consolidación o enfermedad intersticial. No se observa linfadenopatía hiliar o mediastínica. No hay presencia de derrame pleural ni neumotórax.*

*Las porciones visualizadas del abdomen superior, estructuras óseas y tejidos blandos no presentan alteraciones destacables.*    1170

FIG 11F

Config = "Encuentre cualquier mención de nódulos pulmonares en este informe radiológico"    1120

Patient Name: John, Doe
Patient DOB: 23/5/1964

EXAM: CT CHEST WITHOUT CONTRAST

CLINICAL INDICATION: Incidental 8mm nodule found on chest x-ray during pre-operative evaluation.

TECHNIQUE: Non-contrast multidetector helical CT of the chest performed using standard protocol.

FINDINGS:    1190
An 8mm well-circumscribed pulmonary nodule is identified in the right lower lobe, located in the posterior basal segment. The nodule demonstrates homogeneous soft tissue density without calcification or cavitation. No associated ground glass opacity or surrounding infiltrate is present.    1110

The remaining lung parenchyma is clear without evidence of consolidation or interstitial disease. No hilar or mediastinal lymphadenopathy is noted. No pleural effusion or pneumothorax is present.

The visualized portions of the upper abdomen, osseous structures, and soft tissues are unremarkable.

IMPRESSION:
8mm solitary pulmonary nodule in right lower lobe. Given the patient's age and smoking history, short-term follow-up CT in 3 months is recommended according to Fleischner Society guidelines.

Europäisches
Patentamt

European
Patent Office

Office européen
des brevets

# EUROPEAN SEARCH REPORT

**Application Number**

EP 26 15 3848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 11 749 407 B1 (MCNAIR DOUGLAS S [US] ET AL) 5 September 2023 (2023-09-05) * column 6, line 53 - column 70, line 12 * * figure 5A * ----- | 1-15 | INV. G16H50/70 |
| Y | US 2017/357642 A1 (CHAPMAN JEFFREY [US] ET AL) 14 December 2017 (2017-12-14) * paragraph [0017] - paragraph [0033] * * claim 1 * ----- | 1-15 | |
| A | US 2019/188219 A1 (LEBLANC DAVID [CA] ET AL) 20 June 2019 (2019-06-20) * paragraph [0012] - paragraph [0112] * ----- | 1-15 | |

| TECHNICAL FIELDS SEARCHED (IPC) |
|---|
| G16H |

The present search report has been drawn up for all claims

| Place of search | Date of completion of the search | Examiner |
|---|---|---|
| Munich | 9 June 2026 | Rinelli, Pietro |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 26 15 3848

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2026

| Patent document cited in search report | | Publication date | Patent family member(s) | | Publication date |
|---|---|---|---|---|---|
| US 11749407 | B1 | 05-09-2023 | US | 10446273 B1 | 15-10-2019 |
| | | | US | 10854334 B1 | 01-12-2020 |
| | | | US | 10957449 B1 | 23-03-2021 |
| | | | US | 11581092 B1 | 14-02-2023 |
| | | | US | 11749407 B1 | 05-09-2023 |
| | | | US | 11842816 B1 | 12-12-2023 |
| | | | US | 11929176 B1 | 12-03-2024 |
| | | | US | 12488892 B1 | 02-12-2025 |
| | | | US | 2024055127 A1 | 15-02-2024 |
| | | | US | 2025157671 A1 | 15-05-2025 |
| | | | US | 2025166842 A1 | 22-05-2025 |
| US 2017357642 | A1 | 14-12-2017 | US | 2017357642 A1 | 14-12-2017 |
| | | | WO | 2017216642 A2 | 21-12-2017 |
| US 2019188219 | A1 | 20-06-2019 | EP | 3208726 A1 | 23-08-2017 |
| | | | US | 2017242922 A1 | 24-08-2017 |
| | | | US | 2019188219 A1 | 20-06-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUMBRERAS, B.** ; **DONAT, L.** ; **HERNA, I.** ; **HERNANDEZ, M.** *Incidental findings in imaging diagnostic tests: a systematic review*, https://doi.org/10.1259/bjr/98067945

- **NAZI, Z. A.** ; **PENG, W.** *Large Language Models in Healthcare and Medical Domain: A Review. Informatics*, 2024, vol. 11 (3), 57, https://doi.org/10.3390/informatics11030057